Europäisches Patentamt

European Patent Office

Office européen des brevets

(19)

(11) **EP 0 931 051 B1**

(12) **EUROPÄISCHE PATENTSCHRIFT**

(45) Veröffentlichungstag und Bekanntmachung des Hinweises auf die Patenterteilung:
**15.12.1999 Patentblatt 1999/50**

(21) Anmeldenummer: **97942914.9**

(22) Anmeldetag: **11.09.1997**

(51) Int Cl.$^6$: **C07C 67/58**, C07C 69/52, C11C 3/04

(86) Internationale Anmeldenummer:
**PCT/EP97/04978**

(87) Internationale Veröffentlichungsnummer:
**WO 98/12169 (26.03.1998 Gazette 1998/12)**

(54) **VERFAHREN ZUR HERSTELLUNG VON FETTSÄUREESTERN**

METHOD FOR PREPARING FATTY ACID ESTERS

PROCEDE DE PREPARATION D'ESTERS D'ACIDE GRAS

(84) Benannte Vertragsstaaten:
**AT BE CH DE DK ES FI FR GB IT LI NL SE**

(30) Priorität: **19.09.1996 DE 19638460**

(43) Veröffentlichungstag der Anmeldung:
**28.07.1999 Patentblatt 1999/30**

(73) Patentinhaber: **Peter, Siegfried, Prof. Dr.**
**91080 Uttenreuth-Weiher (DE)**

(72) Erfinder:
• **PETER, Siegfried**
**D-91080 Uttenreuth (DE)**
• **GANSWINDT, Ruth**
**D-63571 Gelnhausen (DE)**
• **WEIDNER, Eckhard**
**D-91056 Erlangen (DE)**

(74) Vertreter: **Winter, Brandl & Partner**
**Patent- und Rechtsanwaltskanzlei**
**Alois-Steinecker-Strasse 22**
**85354 Freising (DE)**

(56) Entgegenhaltungen:
**EP-A- 0 416 661**          **US-A- 4 675 132**

• **JACKSON M A ET AL: "METHANOLYSIS OF SEED OILS IN FLOWING SUPERCRITICAL CARBON DIOXIDE" JOURNAL OF THE AMERICAN OIL CHEMISTS' SOCIETY, Bd. 73, Nr. 3, 1.März 1996, Seiten 353-356, XP000580098**
• **GUNNLAUGSDOTTIR H ET AL: "LIPASE-CATALYZED ALCOHOLYSIS OF COD LIVER OIL IN SUPERCRITICAL CARBON DIOXIDE" JOURNAL OF THE AMERICAN OIL CHEMISTS' SOCIETY, Bd. 72, Nr. 4, 1.April 1995, Seiten 399-405, XP000504271**
• **DATABASE WPI Section Ch, Week 9604 Derwent Publications Ltd., London, GB; Class B07, AN 96-036019 XP002050667 -& JP 07 304 977 A (SANEIGEN FFI KK) , 21.November 1995**

## Beschreibung

Die Erfindung betrifft ein Verfahren zur Herstellung von Fettsäureestern und insbesondere ein Verfahren, bei welchem die Fettsäureester durch Umesterung von Fetten und Ölen tierischen oder vegetabilischen Ursprungs erhalten werden.

Die Herstellung von Alkyl- und insbesondere Methylestern durch Alkoholyse von Fetten und Ölen wird neuerdings in Hinblick auf die Erzeugung von Dieselkraftstoffen aus nachwachsenden Rohstoffen lebhaft diskutiert.

Die Glycerinkomponente von Fetten und Ölen läßt sich schon bei niedriger Temperatur gegen einwertige Alkohole niedrigen Molekulargewichts austauschen. Die Alkoholyse wird durch Säuren oder Alkalien katalysiert. In der Technik wird häufig das Bradshaw-Verfahren zur Umesterung von Fetten mit Methanol angewandt (US-A-2,271,619 und 2,360,844). Hierbei wird das Fett, dessen Säurezahl nicht über 1,5 liegen soll, bei 80°C mit einem Überschuß Methanol in Gegenwart von 0,1 bis 0,5 % Natriumhydroxid kurz gerührt. Beim ruhigen Stehen scheidet sich das Glycerin praktisch wasserfrei am Boden des Gefäßes ab.

Das Verfahren ist bemerkenswert nicht nur hinsichtlich der Erzeugung von Methyl- oder Ethylestern direkt aus dem Fett, ohne Zwischenschritt der Hydrolyse, sondern auch wegen der niedrigeren Reaktionstemperatur und weil keine spezielle korrosionsbeständige Ausrüstung erforderlich ist.

Wird Methanol verwendet, verläuft die Reaktion nach folgendem Schema, in dem R einen Fettsäurerest bedeutet:

$$C_3H_5(R)_3 + 3CH_3OH \rightarrow C_3H_5(OH)_3 + 3\ CH_3R$$

Die Reaktion wird in einem offenen Tank ausgeführt, der aus gewöhnlichen Kohlenstoffstahl bestehen kann. Das Fett muß trocken, sauber und vor allem neutral sein. Es wird auf etwa 80°C erhitzt, und kommerzielles wasserfreies Methanol (99,7 %) wird hinzugefügt, in welchem 0,1 bis 0,5 % Natriumhydroxid oder Kaliumhydroxid gelöst ist. Eine Menge an Alkohol, die etwa das 1,6-fache der theoretisch erforderlichen Menge entspricht, wird empfohlen. Jedoch kann der Alkohol auf etwa das 1,2-fache des theoretischen Wertes reduziert werden, wenn der Prozeß in drei Schritten ausgeführt wird. Mehr als das 1,75-fache der theoretischen Menge beschleunigt die Reaktion nicht und beeinträchtigt die anschließende Entfernung des Glycerins durch die Schwerkraft.

Nach Hinzufügen des Alkohols wird das Gemisch einige Minuten lang gerührt und dann in Ruhe stehen gelassen. Das Glycerin beginnt sich ziemlich unmittelbar abzutrennen. Da es praktisch wasserfrei und viel schwerer als die anderen Flüssigkeiten ist, setzt es sich leicht ab, um eine Schicht am Boden des Tanks zu bilden. Die Umsetzung des Öls zu Methylester ist gewöhnlich nach einer Stunde zu 98% vollständig.

Die untere Schicht enthält nicht weniger als 90 % des Glycerins, das ursprünglich im Fett vorhanden war. Die obere Schicht besteht aus Methylestern, den Hauptteilen des nicht reagierten Alkohols und Alkalis, dem Rest des Glycerins und einem sehr kleinen Anteil an Seife. Diese verschiedenen Verunreinigungen werden aus den Estern durch mehrfaches Waschen mit kleinen Mengen warmen Wassers entfernt.

Im Bradshaw-Verfahren werden die erhaltenen Methylester in einem kontinuierlichen Prozeß zur Herstellung von wasserfreier Seife verwendet. Die Ester werden durch Natriumhydroxid oder Kaliumhydroid bei einer niedrigen Temperatur leicht verseift, und das leicht flüchtige Methanol wird freigesetzt und für die Wiederverwendung zurückgewonnen.

Das beschriebene Verfahren sollte sich jedoch auch dazu eignen, Monoester für die Fraktionierung und damit maßgeschneiderte Fette und Öle herzustellen. Die Methyl- und Ethylester der Fettsäuren sind flüssig und relativ stabil, nicht korrosiv und niedrig siedend, und werden deswegen häufig den freien Säuren vorgezogen, insbesondere wenn die Fraktionierung bei erhöhten Temperaturen ausgeführt werden muß, wie dies bei einer Destillation der Fall ist.

Die Umesterung von Erdnussöl mit Ethanol wurde von Feuge und Gros, J. Am. Oil Chem. Soc. 26 (1949) 97-102, im Detail studiert. Sie stellten fest, daß die optimale Temperatur für die Reaktion in der Nachbarschaft von 50°C liegt. Bei dieser Temperatur wurde eine höhere Ausbeute an Glycerin erhalten als bei 30°C oder 70°C.

Bei der beschriebenen Alkoholyse von Triglyceriden entsteht jedoch normalerweise nicht nur freies Glycerin und Monoester, sondern es werden auch Mono- und Diglyceride und Partialester des betreffenden Alkohols gebildet.

Toyama und Mitarbeiter (Y. Toyama, T. Tsuchiya und T. Ishikawa, J. Soc. Chem. Ind. Japan, 36 (1933) 230-232B) zeigten z.B., daß sich in Gegenwart von Natriumhydroxid zwischen Methanol oder Ethanol und Fetten bei Raumtemperatur innerhalb von zwei Stunden Gleichgewicht einstellt. Um die Reaktion bis zur vollständigen Umsetzung des Fettes zum Monoester fortschreiten zu lassen, ist in diesem Verfahren die Abtrennung des freigesetzten Glycerins aus dem Reaktionsgemisch erforderlich.

In einem Überblick von Wright und Mitarbeitern (H.J. Wright, J.B. Segur, H.V. Clark, S.K. Coburn, E.E. Langdon und R.N. DuPuis, Oil & Soap, 21 (1944) 145-148) werden die genauen Bedingungen für die Alkoholyse von Fetten mit Methanol und Ethanol im einzelnen betrachtet. Außerdem wird über Experimente zur Alkoholyse mit anderen Monohydroxyalkoholen berichtet. Es wird darauf hingewiesen, daß die oben beschriebene, durch Alkali katalysierte Alkoholyse nur vollkommen erfolgreich ist, wenn das Fett praktisch frei von freien Fettsäuren und die Reaktionsmischung wasserfrei ist. Wenn eine dieser Bedingungen nicht erfüllt ist, erfolgt Seifenbildung, was zu einem Verlust an Alkalität und dem Aufbau einer Gelstruktur führt, welche die Trennung und das Absetzen des Glycerins verhindert oder verlangsamt. Bei der Ethanolyse treten Schwierigkeiten auf, wenn der Gehalt des Fettes an freien Fettsäuren etwa 0,5 % übersteigt. Wenn 30 Teile Ethanol, 100 Teile Baumwollsaatöl und 0,5 % Natriumhydroxid zur Reaktion gebracht werden, wird die Ausbeute an Glycerin durch 0,3 % Wasser im Reaktionsgemisch merklich vermindert. Jedoch kann die Wirkung von Feuchtigkeit durch den Zusatz von weiterem Alkali und/ oder Alkohol teilweise kompensiert werden. Die Wassertoleranz der obigen Mischung wird auf 0,5 bis 0,6 % erhöht, wenn der Gehalt an Katalysator verdoppelt oder die Menge an Alkohol auf 40 Teile erhöht wird.

Von Wright und Mitarbeitern wurde ebenfalls gezeigt, daß die Geschwindigkeit der Gesamtreaktion prinzipiell von der Zeit begrenzt wird, die zur Abtrennung des Glycerins durch die Schwerkraft erforderlich ist. Eine kontinuierliche Zentrifugalabscheidung bei 65°C mit einer Verweilzeit von etwa 5 Minuten ergab ein ziemlich gutes Ergebnis von etwa 85 % des theoretischen Wertes. Die Behauptung von Bradshaw und Meuly, daß weniger Alkohol bei schrittweiser Zugabe und Glycerinabtrennung benötigt wird, wurde im Fall der Methanolyse bestätigt, jedoch nicht im Fall der Ethanolyse, weil diese Methode Gelbildung verursacht.

Insbesondere wenn Natrium und Kalium als Katalysatoren verwendet werden, treten bei der Umsetzung der Triglyceride mit Methanol und Ethanol verschiedene Probleme auf. Der auf beide Phasen verteilte Katalysator muß nach Abschluß der Reaktion entfernt werden. Die Trennung der beiden Phasen nach der Reaktion verläuft so langsam, daß große Reaktionsvolumina erforderlich werden. Im Monoester sind noch sehr feine Glycerintröpfchen suspendiert, die mit Wasser ausgewaschen werden müssen. Je nach weiterer Verwendung des Glycerins ist es erforderlich, den gelösten Katalysator zu entfernen. Die erforderliche Auftrennung der bei der Reaktion gebildeten Emulsion ist äußerst aufwendig. Ein weiteres Problem wird darin gesehen, daß die Reaktion zuweilen nicht sogleich anspringt.

Wegen der beschriebenen Nachteile ist das obige Verfahren in der Praxis zur Herstellung von Fettsäureestern oft schlecht anwendbar.

US-A-4 675 132 offenbart ein Verfahren zum Aufkonzentrieren von mehrfach ungesättigten Fettsäuren aus Fischölen, worin die Fettsäureglyceride mit einem niederen Alkohol unter Bildung einer Mischung aus Fettsäureniederalkylestern umgeestert werden und diese Ester mit überkritischem Kohlendioxid extrahiert werden.

Im Journal of the American Oil Chemists' Society, Bd. 72, Nr. 4, 1995, Seiten 399-405, wird ein Verfahren zur enzymatischen Alkoholyse von Lebertran in überkritischem Kohlendioxid unter Verwendung einer immobilisierten Lipase beschrieben. Im Journal of the American Oil Chemists' Society, Bd. 73, Nr. 3, 1996, Seiten 353-356, wird die enzymatische Methanolyse von Öl aus Ölsaaten in überkritischem Kohlendioxid unter Verwendung einer immobilisierten Lipase beschrieben.

Aufgabe der Erfindung ist es, ein Verfahren zur Herstellung von Fettsäureestern aus Fettsäuretriglyceriden anzugeben, welches einfach und kostengünstig durchzuführen ist und eine möglichst hohe Ausbeute an dem gewünschten Reaktionsprodukt liefert. Das Verfahren sollte außerdem eine kontinuierliche Reaktionsführung bei möglichst kleinen Reaktionsvolumina erlauben.

Diese Aufgabe wird durch das Verfahren gemäß Anspruch 1 gelöst. Weitere Ausführungsformen ergeben sich aus den Unteransprüchen.

Es wurde überraschenderweise gefunden, daß die Abtrennung des Glycerins aus dem Reaktionsgemisch dadurch erheblich beschleunigt werden kann, daß der gebildete Fettsäureester mit einem nahekritischem Extraktionsmittel aus der Reaktionsmischung extrahiert wird. Dabei wird die Reaktionsgeschwindigkeit nur unbedeutend verlangsamt. Läßt man einen flüssigen Strom des Extraktionsmittels durch die Reaktionsmischung streichen, wird die Phasentrennung beschleunigt und gleichzeitig der entstehende Fettsäureester aus dem Reaktionsgemisch entfernt. Da die entstehenden Fettsäureester im Extraktionsmittel viel leichter löslich sind als Tri- und Partialglyceride, wird bei geeigneten Bedingungen ein reines Produkt unmittelbar erhalten. Die Beschleunigung der Phasentrennung, welche der kritische Faktor bei der Umesterungsreaktion ist, gelingt unabhängig davon, ob die Reaktion mit Hilfe homogener oder heterogener Katalysatoren beschleunigt wird. In beiden Fällen werden Emulsionen gebildet, deren Spaltung bisher ausgesprochen aufwendig war.

Als Ausgangsverbindungen des erfindungsgemäßen Verfahrens können grundsätzlich alle Fettsäure-Triglyceride eingesetzt werden. Besonders bevorzugt dienen als Ausgangsstoffe tierische oder vegetabilische Fette oder Öle, vor allem solche mit 10 bis 22 Kohlenstoffatomen und insbesondere 12 bis 18 Kohlenstoffatomen im Fettsäurerest. Beispielhaft für vegetabilische Ausgangsstoffe können Sojabohnen-, Raps-, Sonnenblumen-, Erdnuß-, Baumwoll-, Palm-, Lein-, Rizinus-, Rüb- und Olivenöl genannt werden.

Das erfindungsgemäße Verfahren eignet sich besonders für die Umesterung von Fettsäuretriglyceriden mit einem einwertigen niedrigen Alkohol, welcher 1 bis 6 Kohlenstoffatome aufweist. Besonders bevorzugt sind Alkohole mit 1 bis 4 Kohlenstoffatomen und insbesondere mit Methanol oder Ethanol. Die Umesterungsreaktion kann grundsätzlich nach den im Stand der Technik bekannten Verfahren erfolgen. Zweckmäßig erfolgt die Umesterung bei einer Temperatur zwischen 20 und 100°C, vorzugsweise bei 30 bis 80°C und insbesondere bei einer Temperatur zwischen 50 und 75°C.

Die Umsetzung der Ausgangsstoffe mit dem einwertigen niederen Alkohol findet vorzugsweise bei einem Druck zwischen Umgebungsdruck und dem Dampfdruck des eingesetzten Alkohols statt.

Die Umesterung kann sowohl in Gegenwart eines homogenen als auch eines heterogenen Katalysators erfolgen. Als homogener Katalysator eignet sich insbesondere ein Alkali. Beispielsweise können alle bereits im Stand der Technik als Katalysatoren für diese Reaktion verwendeten Alkylate eingesetzt werden. Beispielhaft können die Natrium- oder Kaliumalkoholate des jeweils bei der Umesterung eingesetzten einwertigen Alkohols genannt werden. Natriumhydrid ist ebenfalls geeignet.

Heterogene Katalysatoren haben gegenüber den homogenen Katalysatoren den Vorteil, daß sie aus der Reaktionsmischung leichter abgetrennt werden können. Im Rahmen der Erfindung wurde ebenfalls nach geeigneten Katalysatoren gesucht. Dabei wurde gefunden, daß saure oder alkalische Ionenaustauscherharze und insbesondere stark alkalische Ionenaustauscherharze als Katalysatoren bei der Umesterung von Fettsäure-Triglyceriden mit einwertigen Alkoholen gut geeignet sind. Als besonders geeignet erwies sich Amberlite® IRA900C1 (Röhm & Haas, Darmstadt), welcher vor seinem Einsatz von der Chlorid- in die Hydroxydform überführt wird. Dies kann beispielsweise durch Ionenaustausch mit 4 % Sodalösung und Natronlauge geschehen. Nach Trocknung mit wasserfreiem Methanol wird ein stark basischer Ionenaustauscher auf Kunstharzbasis (Polystyrol) erhalten. Der so erhaltene Katalysator ergab in der Regel nach 30minütiger Reaktion eine Umsetzung von 80 % der Theorie.

Als Extraktionsmittel wird erfindungsgemäß ein sogenanntes Niederdruckgas (low pressure gas) eingesetzt. Unter einem Niederdruckgas wird hier ein Gas verstanden, welches eine reduzierte Temperatur bei 20°C von etwa 0,7 aufweist, vorzugsweise von größer oder gleich 0,7. Beispiele solcher Niederdruckgase sind Kohlendioxid, Propan, Butan, Dimethylether, Ethylacetat und andere. Es können auch Mischungen von wenigstens zwei dieser Verbindungen eingesetzt werden. Das erfindungsgemäße Verfahren wird derart durchgeführt, daß das Extraktionsmittel in nahekritischem Zustand im Reaktionsgemisch gelöst wird. Die gewünschte Dichte des Extraktionsmittels wird durch geeignete Wahl von Temperatur und Druck einreguliert. Zweckmäßig ist die Dichte des Extraktionsmittels wenigstens gleich der kritischen Dichte. Die Temperatur, bei der extrahiert wird, liegt zwischen 0 und 200°C, vorzugsweise zwischen 30 und 100°C. Bevorzugte Extraktionsmittel sind Kohlendioxid, Propan, Butan oder Dimethylether. Propan, Butan und Dimethylether sind besonders bevorzugt.

Zweckmäßig wird mit der Extraktion des gebildeten Fettsäureesters begonnen, wenn das eingesetzte Fettsäuretriglycerid zu wenigstens 80 % und insbesondere zu 80 - 95 % umgesetzt ist.

Eine bevorzugte Form der Durchführung des erfindungsgemäßen Verfahrens besteht darin, die zu trennende Emulsion, die sich im Verlauf der Reaktion gebildet hat, in eine Extraktionskolonne, vorzugsweise in den mittleren Teil einer Gegenstrom-Extraktionskolonne, einzuspeisen und im Gegenstrom mit dem nahekritischen Extraktionsmittel in Kontakt zu bringen. Das die Kolonne von unten nach oben durchstreichende Extraktionsmittel löst bevorzugt die Alkylester, die die höchste Löslichkeit der in der Reaktionsmischung befindlichen Verbindungen besitzen. Die nicht gelösten Anteile des Zulaufs fließen in der Kolonne nach unten. Dabei verarmt die Reaktionsmischung zunehmend an Alkylestern, bis schließlich im Sumpf der Gegenstromkolonne ein Produkt anfällt, das aus Glycerin besteht, in welchem noch geringe Verunreinigungen an Partialglyceriden und Triglyceriden gelöst sind. Wird die Alkoholyse mit Hilfe von homogenen Katalysatoren durchgeführt, so werden diese ebenfalls mit dem Sumpfprodukt abgeschieden. Heterogener Katalysator wird zweckmäßig bereits vor der Überführung der Reaktionsmischung in die Gegenstromextraktionskolonne abgetrennt.

Der den Kopf der Extraktionskolonne verlassende Extrakt wird zweckmäßig einer weiteren Kolonne zugeführt, in der durch Temperaturerhöhung und/oder Druckverminderung die Dichte des Extraktionsmittels soweit herabgesetzt wird, daß die extrahierten Fettsäureester quantitativ ausfallen. Besonders vorteilhaft läßt sich die vollständige Trennung von Extraktionsmittel und Extrakt durch Rektifikation des Kopfprodukts der Extraktionskolonne durchführen.

Die Rektifikation erfolgt vorzugsweise unter einem Druck, der nur wenig geringer ist als der Druck in der Extraktionskolonne. Günstig ist weiterhin, einen Teil der am Sumpf der Rektifikationskolonne in großer Reinheit anfallenden Fettsäureester als Rücklauf auf den Kopf der Extraktionskolonne zurückzupumpen.

Das Sumpfprodukt der Extraktionskolonne enthält das entstandene Glycerin und im allgemeinen vernachlässigbare Verunreinigungen an Partialglyceriden und nicht abreagierten Triglyceriden. Bei homogener Katalyse enthält das Sumpfprodukt den Katalysator, der in einem weiteren Prozeßschritt entfernt werden kann. Dies kann beispielsweise durch Destillation geschehen. Im Falle einer heterogenen Katalyse erhält man ein Glycerin, das wegen seiner Reinheit unmittelbar einer weiteren Verwendung zugeführt werden kann.

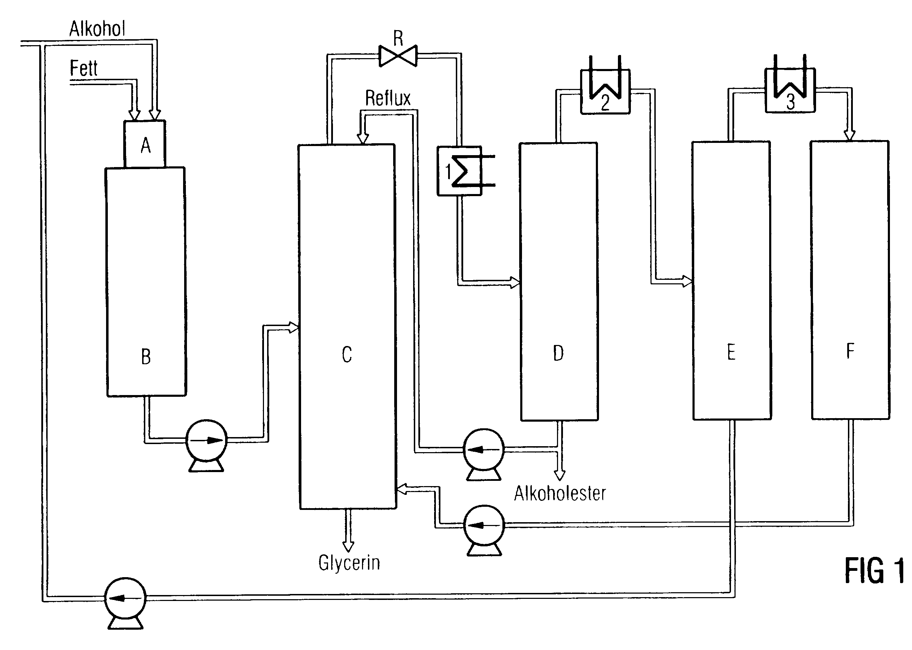

Das Verfahren wird im folgenden unter Bezugnahme auf eine Zeichnung näher erläutert. Fig. 1 zeigt schematisch eine Vorrichtung zur Durchführung des erfindungsgemäßen Verfahrens.

Die Vorrichtung umfaßt einen Reaktor B, in dem die Reaktion des Ausgangsprodukts, Fettsäuretriglycerid, mit dem Alkohol erfolgt. Alkohol und Triglycerid werden dem Reaktionsgefäß B über einen Inline-Mischer A zugepumpt. Im Fall einer homogenen Katalyse wird der Katalysator zweckmäßig zuvor dem Alkohol zugemischt. Der heterogene Katalysator wird vorzugsweise in den Reaktor gegeben. Das Reaktionsgefäß wird vom Reaktionsgemisch langsam durchströmt. Die Abmessungen des Reaktionsgefäßes werden zweckmäßig so gewählt, daß die Verweilzeit der Reaktionsmischung im Reaktionsgefäß ausreicht, um bei einer Temperatur im Bereich von 20 bis 100°C einen Umsatz von wenigstens 80 % und insbesondere zwischen 80 und 90 %, der Theorie zu erhalten. Die den Reaktor B verlassende Reaktionsmischung besteht im wesentlichen aus Glycerin und Fettsäureester. Sie wird dem mittleren Teil eines Extraktors C zugepumpt.

Der Druck im Extraktor C ist höher als der Dampfdruck des im erfindungsgemäßen Verfahren verwendeten Extraktionsmittels. Die Temperatur im Extraktor C liegt im Bereich vom 20 bis 200°C. Das Extraktionsmittel wird dem Extraktor C am Boden zugeführt und durchströmt den Extraktor von unten nach oben im Gegenstrom zum Reaktionsgemisch. Dabei wird zunehmend Fettsäureester aus dem Reaktionsgemisch herausgelöst, und die Emulsion trennt sich auf.

Bei heterogener Katalyse wird man den Teil des Extraktors C unterhalb der Zuführung des Reaktionsgemisches mit dem (pellierten) Katalysator füllen. Auf diese Weise können durch Nachreaktion praktisch 100 % der eingesetzten Triglyceride umgesetzt werden.

Am Kopf des Extraktors verläßt das mit Fettsäureester beladene Extraktionsmittel den Extraktor und wird im Entspannungsventil R auf den Druck in der Rektifikationskolonne D entspannt. Nach dem Passieren eines Wärmeaustauschers 1 wird das beladene Extraktionsmittel in den mittleren Teil einer Rektifikationskolonne D eingespeist. Das Lösemittel, welches aus überschüssigem Alkohol und dem Extraktionsmittel besteht, wird durch Rektifikation unter Druck von den extrahierten Substanzen quantitativ abgetrennt. Als Sumpfprodukt der Rektifikationskolonne wird lösemittelfreier Fettsäureester abgezogen.

Das die Rektifikationskolonne verlassende dampfförmige Gemisch aus Alkohol und Extraktionsmittel wird im Wärmetauscher 2 soweit abgekühlt, daß der Alkohol auszukondensieren beginnt.

Im Kondensator E werden durch Rektifikation bei konstantem Druck Alkohol und Extraktionsmittel voneinander getrennt. Am Sumpf der Kolonne E wird der erhaltene Alkohol abgezogen und in den Inline-Mischer A am Beginn des Prozeß zurückgepumpt. Das die Kolonne E am Kopf verlassende Extraktionsmittel wird im Wärmetauscher 3 weiter gekühlt und in den flüssigen Zustand überführt. Das im Kondansator F niedergeschlagene Extraktionsmittel wird in den Sumpf des Extraktors C zurückgepumpt.

Die unter Bezugnahme auf die Zeichnung beschriebene Ausführungsform stellt eine besonders effektive Variante der Ausführung des erfindungsgemäßen Verfahrens dar. Die Erfindung wird durch die folgenden Beispiele weiter erläutert. Die Prozentangaben sind in Gew.-%.

Beispiel 1

In einen Reaktionsautoklaven aus Edelstahl wurden 200 g Schlachtfett und das 1,2-fache der theoretisch benötigten Menge an Methanol gegeben. Danach wurde auf 75°C erwärmt und 2 g Natriummethylat als Katalysator hinzugefügt. Nach einer Zeit von 2,5 Minuten kräftigen Rührens bei Umgebungsdruck wurden vom Reaktionsgemisch Proben entnommen und analysiert. Etwa 94 % der eingesetzten Menge an Fett waren in Methylester umgesetzt. Nach Unterbrechung des Rührvorganges wurde flüssiges Propan bei einem Druck von 35 bar durch die Reaktionsmischung geleitet. Nach Verlassen des Autoklaven wurde das Propan entspannt und der im Propan gelöste Extrakt in einem Probefinger aufgefangen. Der Extrakt hatte abzüglich des noch in geringer Konzentration vorhandenen Propans 87 Gew.-% Methylester und 10 Gew.-% Methanol. Der Rest bestand aus Triglyceriden, Partialglyceriden und etwas Glycerin.

Beispiel 2

In einem Autoklaven von 1 Liter Inhalt wurden 200 g Rüböl und das 1,5-fache an Methanol eingefüllt. Danach wurde auf 65°C erwärmt und 2 g Natriummethylat hinzugefügt. Bei Umgebungsdruck wurde 5 Minuten lang kräftig gerührt. Nach Beendigung des Rührvorgangs wurden von dem als Emulsion vorliegenden Reaktionsgemisch Proben entnommen und analysiert. Etwa 91 % des Ausgangsproduktes waren in Methylester umgesetzt worden. Im Anschluß daran ließ man Dimethylether bei einem Druck von 30 bar durch den Autoklaven von unten nach oben strömen. Nach Verlassen des Autoklaven wurde der Dimethylether auf Umgebungsdruck entspannt. Dabei fiel der Extrakt aus und wurde in einem Kühlfinger gesammelt. Der Extrakt enthielt abzüglich des noch geringfügig vorhandenen Dimethylethers etwa 60 % Methylester und 35 % Methanol. Der Rest bestand aus Triglyceriden, Partialglyceriden und etwas Glycerin.

Beispiel 3

200 g Rüböl und 150 g des festen basischen Katalysators Amberlite® IRA 900 wurden in einem Autoklaven von 1 Liter Inhalt eingefüllt. Nach Erwärmen auf 68°C wurde Methanol bis zum Erreichen des Siededrucks zugegeben. Etwa 30 Minuten wurde der Autoklav vorsichtig geschüttelt und anschließend der Katalysator von dem Reaktionsgemisch abfiltriert. Die Analyse ergab, daß 80 % des eingesetzten Rüböls zu Methylester umgesetzt worden waren.

Beispiel 4

200 g raffiniertes Kokosfett und 200 g des basischen Katalysators Amberlite® IRA 900 wurden in einem Autoklaven von 1 Liter Inhalt eingefüllt. Nach Erwärmen auf 71°C wurde Methanol bis zum Erreichen des Siededrucks (etwa 1,05 atm.) zugepumpt. Nach 2 Stunden wurde das Extraktionsgemisch abfiltriert und nach Separation der Glycerinphase die Ölphase analysiert. Die Ölphase hatte folgende Zusammensetzung: Methylester 92,3 Gew.-%; Diglyceride 6,6 Gew.-% ; Glycerin 1,1 Gew.-%.

**Patentansprüche**

1. Verfahren zur Herstellung von Fettsäureestern, worin Fettsäuretriglycerid in Gegenwart eines homogenen oder heterogenen Katalysators mit einwertigem Alkohol mit 1 bis 6 Kohlenstoffatomen umgeestert wird, dadurch gekennzeichnet, daß der gebildete Fettsäureester mit einem nahekritischen Extrakrionsmittel aus der Reaktionsmischung extrahiert wird, mit der Maßgabe, daß im Falle eines aus Kohlendioxid bestehenden Extraktionsmittels nicht im überkritischen Bereich extrahiert wird.

2. Verfahren gemäß Anspruch 1, dadurch gekennzeichnet, daß das Extraktionsmittel ein Gas mit einer reduzierten Temperatur bei 20°C von etwa 0,7 ist, vorzugsweise von größer oder gleich 0,7.

3. Verfahren gemäß Anspruch 1 oder Anspruch 2, dadurch gekennzeichnet, daß als Extraktionsmittel Kohlendioxid, Propan, Butan, Dimethylether, Ethylacetat oder eine Mischung von wenigstens zweien dieser Verbindungen eingesetzt wird.

4. Verfahren gemäß Anspruch 3, dadurch gekennzeichnet, daß das Extraktionsmittel mit einer Dichte von wenigstens gleich der kritischen Dichte bei einer Temperatur zwischen 0°C und 200°C, vorzugsweise bei 30 bis 100°C, eingesetzt wird.

5. Verfahren gemäß Anspruch 4, dadurch gekennzeichnet, daß als Extraktionsmittel Propan oder butan oder Dimethylether verwendet wird.

6. Verfahren gemäß einem der Ansprüche 1 bis 5, dadurch gekennzeichnet, daß als einwertiger Alkohol ein Alkohol mit 1 bis 4 Kohlenstoffatomen, insbesondere Methanol oder Ethanol, eingesetzt wird.

7. Verfahren gemäß einem der Ansprüche 1 bis 6, dadurch gekennzeichnet, daß als Fettsäuretriglycerid tierische oder vegetabilische Fette oder Öle, insbesondere solche mit 10 bis 22 und vorzugsweise 12 bis 18 Kohlenstoffatomen im Fettsäurerest, eingesetzt werden.

8. Verfahren gemäß einem der Ansprüche 1 bis 7, dadurch gekennzeichnet, daß als heterogener Katalysator ein Ionenaustauschharz und insbesondere ein stark alkalisches Ionenaustauscherharz eingesetzt wird.

9. Verfahren gemäß einem der Ansprüche 1 bis 7, dadurch gekennzeichnet, daß als homogener Katalysator ein Alkali eingesetzt wird.

10. Verfahren gemäß einem der Ansprüche 1 bis 9, dadurch gekennzeichnet, daß die Umesterung bei einer Temperatur von 20 bis 100°C, vorzugsweise 30 bis 80°C und insbesondere 50 bis 75°C, erfolgt.

11. Verfahren gemäß einem der Ansprüche 1 bis 10, dadurch gekennzeichnet, daß die Umesterung bei einem Druck zwischen Umgebungsdruck und dem Dampfdruck des einwertigen Alkohols erfolgt.

12. Verfahren gemäß einem der Ansprüche 1 bis 1i, dadurch gekennzeichnet, daß mit der Extraktion begonnen wird, nachdem das eingesetzte Fettsäuretriglycerid zu wenigstens 80 % und insbesondere zu 80 bis 95 % umgesetzt ist.

13. Verfahren gemäß einem der Ansprüche 1 bis 12, dadurcn gekennzeichnet, daß die Extraktion in einer Gegenstromkolonne (C) erfolgt, der das Extraktionsmittel in einem unteren Bereich und die zu extrahierende, den gebildeten Fettsäureester umfassende Reaktionsmischung einem mittleren Bereich der Gegenstromkolonne (C) zugeführt wird und das mit Fettsäureester und Alkohol angereicherte Extraktionsmittel am Kopf der Kolonne und das von Fettsäureester im wesentlichen befreite Glycerin am Bcden der Kolonne abgezogen werden.

14. Verfahren gemäß Anspruch 13, dadurch gekennzeichnet, daß das mit Fettsäureester und Alkohol angereicherte Extraktionsmittel in einer Rektifikationskolonne (D) durch Druckverminderung und/oder Temperaturerhöhung von Fettsäureester befreit wird und Fettsäureester am Boden und mit Alkohol angereichertes Extraktionsmittel am Kopf der Rektifikationskolonne abgezogen werden.

15. Verfahren gemäß Anspruch 14, dadurch gekennzeichnet, daß der Alkohol in einem Kondensator (E) vom Extraktionsmittel getrennt und gegebenenfalls der weiteren Umsetzung mit Fettsäuretriglycerid zugeführt wird.

16. Verfahren gemäß Anspruch 15, dadurch gekennzeichnet, daß das von Alkohol befreite Extraktionsmittel in einem Kondensator (F) kondensiert und gegebenenfalls zur Extraktionskolonne (C) zurückgeführt wird.

## Claims

1. A method for producing fatty acid esters, wherein fatty acid triglyceride in the presence of a homogeneous or heterogeneous catalyst is transesterified with a monovalent alcohol having 1 to 6 carbon atoms, characterized in that the formed fatty acid ester is extracted from the reaction mixture by means of a near-critical extractant, provided that in the case of an extractant consisting of carbon dioxide the extraction is not carried out under supercritical conditions.

2. The method according to claim 1, characterized in that said extractant is a gas having a reduced temperature at 20°C of about 0.7, preferably of equal to or greater than 0.7.

3. The method according to claim 1 or claim 2, characterized in that carbon dioxide, propane, butane, dimethyl ether, ethyl acetate or a mixture of at least two of these compounds is employed as the extractant.

4. The method according to claim 3, characterized in that said extractant is employed at a density which is at least equal to the critical density and at a temperature between 0°C and 200°C, preferably at 30 to 100°C.

5. The method according to claim 4, characterized in that propane or butane or dimethyl ether is employed as the extractant.

6. The method according to any one of claims 1 to 5, characterized in that an alcohol having 1 to 4 carbon atoms, in particular methanol or ethanol, is employed as the monovalent alcohol.

7. The method according to any one of claims 1 to 6, characterized in that animal or vegetable fats or oils are employed as the fatty acid triglyceride, in particular those comprising 10 to 22 and preferably 12 to 18 carbon atoms in the fatty acid radical.

8. The method according to any one of claims 1 to 7, characterized in that an ion exchange resin, and in particular a strongly alkaline ion exchange resin, is employed as the heterogeneous catalyst.

9. The method according to any one of claims 1 to 7, characterized in that an alkali is employed as the homogeneous catalyst.

10. The method according to any one of claims 1 to 9, characterized in that transesterification takes place at a temperature of 20 to 100°C, preferably 30 to 80°C, and in particular 50 to 75°C.

11. The method according to any one of claims 1 to 10, characterized in that transesterification takes place at a pressure between ambient pressure and the vapor pressure of the monovalent alcohol.

12. The method according to any one of claims 1 to 11, characterized in that extraction is started after the employed fatty acid triglyceride is converted by at least 80%, and in particular by 80 to 95%.

13. The method according to any one of claims 1 to 12, characterized in that extraction takes place in a countercurrent column (C) to which the extractant is supplied in a lower region thereof and to which the reaction mixture to be extracted, comprising the formed fatty acid ester, is supplied to a middle region of the countercurrent column (C), and the extractant enriched with fatty acid ester and alcohol is withdrawn at the top of the column, and the glycerol substantially freed from fatty acid ester is withdrawn at the bottom of the column.

14. The method according to claim 13, characterized in that the extractant enriched with fatty acid ester and alcohol is freed from fatty acid ester in a rectifying column (D) by reduced pressure and/or increased temperature, and fatty acid ester is withdrawn at the bottom and extractant enriched with alcohol is withdrawn at the top of the rectifying column.

15. The method according to claim 14, characterized in that the alcohol is separated from the extractant in a condenser (E) and optionally supplied to further conversion with fatty acid triglyceride.

16. The method according to claim 15, characterized in that the extractant freed from alcohol is condensed in a condenser (F) and optionally recycled to the extraction column (C).

## Revendications

1. Procédé pour la fabrication d'esters d'acide gras, dans lequel du triglycéride d'acide gras est transestérifié en présence d'un catalyseur homogène ou hétérogène avec un alcool monovalent présentant 1 à 6 atomes de carbone, caractérisé en ce que l'ester d'acide gras formé est extrait du mélange de réaction moyennant un moyen d'extraction quasi-critique, à la condition que si le moyen d'extraction est un dioxyde de carbone, l'extraction ne soit pas faite dans la plage surcritique.

2. Procédé selon la revendication 1, caractérisé en ce que le moyen d'extraction est un gaz possédant, à une température de 20°C, une température réduite de 0,7 environ, de préférence supérieure ou égale à 0,7.

3. Procédé selon la revendication 1 ou la revendication 2, caractérisé en ce qu'on utilise comme moyen d'extraction du dioxyde de carbone, du propane, du butane, du diméthylether, de l'éthylacétate ou un mélange d'au moins deux de ces composés.

4. Procédé selon la revendication 3, caractérisé en ce que le moyen d'extraction est utilisé à une densité au moins égale à la densité critique, à une température entre 0°C et 200°C, de préférence de 30 à 100°C.

5. Procédé selon la revendication 4, caractérisé en ce qu'on utilise comme moyen d'extraction du propane ou du butane ou du diméthylether.

6. Procédé selon l'une quelconque des revendications 1 à 5, caractérisé en ce qu'on utilise comme alcool monovalent un alcool présentant 1 à 4 atomes de carbone, notamment du méthanol ou de l'éthanol.

7. Procédé selon l'une quelconque des revendications 1 à 6, caractérisé en ce qu'on utilise comme triglycéride d'acide gras des graisses ou des huiles animales ou végétales, notamment celles qui présentent 10 à 22 et de préférence 12 à 18 atomes de carbone dans le reste d'acide gras.

8. Procédé selon l'une quelconque des revendications 1 à 7, caractérisé en ce qu'on utilise comme catalyseur hétérogène une résine d'échange d'ions et notamment une résine d'échange d'ions fortement alcaline.

9. Procédé selon l'une quelconque des revendications 1 à 7, caractérisé en ce qu'on utilise comme catalyseur homogène un alcali.

10. Procédé selon l'une quelconque des revendications 1 à 9, caractérisé en ce que la transesterification est faite à une température de 20 à 100°C, de préférence à une température de 30 à 80°C et notamment de 50 à 75°C.

11. Procédé selon l'une quelconque des revendications 1 à 10, caractérisé en ce que la transesterification est faite à une pression entre la pression ambiante et la pression de vaporisation de l'alcool monovalent.

12. Procédé selon l'une quelconque des revendications 1 à 11, caractérisé en ce qu'on commence l'extraction après que le triglycéride d'acide gras utilisé soit transformé à 80 % au moins et notamment à 80 % jusqu'à 95 %.

13. Procédé selon l'une quelconque des revendications 1 à 12, caractérisé en ce que l'extraction se fait dans une colonne à courant contraire (C) à laquelle le moyen d'extraction est amené dans une zone inférieure et le mélange de réaction à extraire, comportant l'ester d'acide gras formé, est amené dans une zone médiane de la colonne à courant contraire (C) et que le moyen d'extraction enrichi de l'ester d'acide gras et d'alcool est retiré à la tête de la colonne, et la glycérine substantiellement libérée de l'ester d'acide gras est retirée au fond de la colonne.

14. Procédé selon la revendication 13, caractérisé en ce que le moyen d'extraction enrichi d'ester d'acide gras et d'alcool est libéré d'esters d'acide gras dans une colonne de rectification (D) par abaissement de la pression et/ou augmentation de la température et que des esters d'acide gras sont retirés au fond et le moyen d'extraction enrichi d'alcool à la tête de la colonne de rectification.

15. Procédé selon la revendication 14, caractérisé en ce que l'alcool est séparé du moyen d'extraction dans un condenseur (E) et, le cas échéant, amené à la transformation ultérieure avec du triglycéride d'acide gras.

16. Procédé selon la revendication 15, caractérisé en ce que le moyen d'extraction libéré de l'alcool est condensé dans un condenseur (F) et est ramené le cas échéant à la colonne d'extraction (C).

FIG 1